# EUROPEAN PATENT SPECIFICATION

(11) **EP 1 687 628 B1**
(45) Date of publication and mention of the grant of the patent: **26.01.2011**
(21) Application number: 04819165.4
(22) Date of filing: 15.11.2004
(51) Int. Cl.: G01N 33/48

(54) **FLUID SAMPLE ANALYSIS DEVICE WITH SEALABLE SAMPLE STORAGE RESERVOIR**
FLÜSSIGPROBENANALYSEVORRICHTUNG MIT VERSCHLIESSBAREM PROBENAUFBEWAHRUNGSRESERVOIR
DISPOSITIF D'ANALYSE D'ECHANTILLON DE FLUIDE A RESERVOIR DE STOCKAGE D'ECHANTILLON HERMETIQUE

(30) Priority: 14.11.2003 US 520437 P
(43) Date of publication of application: 09.08.2006
(73) Proprietor: Alere Switzerland GmbH, 6300 Zug (CH)
(72) Inventor: TUNG, Hsiaoho Edward, San Diego, CA 92130 (US); WU, Yuzhang, Hangzhou 310023 (CN); DAI, Jielin, Hangzhou 310023 (HK); YANG, Ying, Hangzhou 310023 (CN)
(74) Representative: Lee, Nicholas John
(86) International application number: PCT/US2004/038426
(87) International publication number: WO 2005/050166

(56) References cited:
- WO-A-01/49820
- WO-A-02/04941
- US-A- 5 965 453
- US-A1- 2003 190 259

## Description

### Field of the Invention

The present invention is directed to devices for the collection and rapid analysis of fluids for analytes of interest.

### Background of the Invention

The following Background of the Invention is intended to aid the reader in understanding the invention and is not admitted to be prior art.

Illicit drug use is an established and growing problem in our society. In 2003, the US Department of Health and Human Services found that an estimated 19.5 million Americans or 8.2 percent of the population aged 12 or older, were current illicit drug users. Current illicit drug use means use of an illicit drug during the month prior to the US Department of Health and Human Services survey interview.
Marijuana was found to be the most commonly used illicit drug, with a rate of 6.2 percent (14.6 million). An estimated 2.3 million persons (1.0 percent) were current cocaine users, 604,000 of whom used crack. Hallucinogens were used by 1.0 million persons, and there were an estimated 119,000 current heroin users.

To combat and monitor this problem, drug testing has become standard procedure in a variety of settings, such as employment, school, sports, law enforcement, and the like. To facilitate this effort, a drug-testing industry has emerged. This industry provides a variety of drug testing products. A typical product is a urine collection cup incorporating analysis tests. These devices can be complicated and difficult or messy to use, or they may pose special problems of sample adulteration by the subject trying to hide their recent drug abuse. In addition, urine samples cannot be collected in certain situations, such as on the road side or in public.

There is therefore a need for better methods and apparatuses for performing sample collection and testing.
US2003/0190259 discloses a multi-chamber container for sampling, storing and preserving for later testing a sample. A swab is used to collect a sample and is inserted into the container, causing the sample to be diverted into each chamber and sealing the container. WO 02/04941 discloses an apparatus which permits the on-line quantitative and quantitative analysis of biofilm and organic and inorganic contaminants which absorb onto the surfaces of paper making equipment.

### Summary of the Invention

In a first aspect, the present invention provides a test device for detecting an analyte suspected of being present in a fluid sample, comprising:
a casing, comprising a port for a sample collection well;
a test compartment containing at least one test element;
a sample collection well situated in the port and comprising an upper chamber, an expression plate, a lower chamber, and a sample outlet, characterised by
a reservoir within or below the lower chamber for storing fluid sample for confirmation testing;
a plunger comprised within the sample collection well, the plunger having a raised position and a lower position;
a seal that fits sealably over the reservoir when the plunger is located in the lower position;
a cap for the sample collection well, wherein a portion of the cap interacts with the plunger to move the plunger from the raised position to the lower position when the cap is applied to the sample collection well,
wherein, when the plunger is in the raised position, the test compartment and lower chamber are in fluid communication and, when the plunger is in the lower position, the test compartment and the lower chamber are not in fluid communication.

The present invention is directed to devices for collecting liquid samples and simultaneously testing the sample for the presence of an analyte of interest or a physical property. In one embodiment the devices are for detecting analytes of interest in body fluids, such as oral fluid or saliva. The sample outlet may provide fluid communication with the test compartment. In one embodiment, the seal is located on the lower end of the plunger. When the plunger is moved from the raised position to the lower position, a reservoir located in fluid communication with the lower chamber of the sample collection well is sealed, thereby preserving a fluid sample for confirmation testing. After the plunger is in the lower position, the reservoir is no longer in fluid communication with the reservoir. In embodiments where a screw cap is used, the plunger is lowered by screwing on the cap, a portion of which interacts with the plunger or a piece connected thereto, and forces the plunger into the lower position.

In a second aspect, the present invention provides kit comprising a test device of the present invention and a sample applicator.
In a third aspect, the present invention provides a method of detecting an analyte suspected of being present in a liquid sample, comprising
applying a liquid sample suspected of containing the analyte to a sample applicator;
applying the liquid sample to a test device of the invention by wringing the sample applicator into the test device; and
determining if the analyte is present in the sample.
Preferred features of each aspect of the invention are set out in the dependent claims herein.

The term "sample reservoir" refers to a sealable area of the apparatus in which fluid sample is stored and preserved from drying out or from accidental contamination. The fluid sample can be stored for confirmatory testing at a later time. In one embodiment, the sample reservoir is an indentation, which can be located in the lower chamber of the sample collection well (e.g., on the bottom of the sample collection well). The term "fluid communication" refers to the ability for liquid to flow and be transmitted between two areas. Thus, the sample collection well and the test compartment are in fluid communication when fluid is able to flow from the sample collection well and through the sample outlet and into the test compartment. "Port" refers to the portion of the casing where the sample collection well interfaces with the casing, and can be placed into fluid communication with the test compartment. The sample collection well can be inserted into the port as a separate part, or the sample collection well and casing can be manufactured as a single part. The sample collection well itself can be made of one part, or assembled from sub-parts. By "fits sealably" is meant that the seal prevents fluid from entering or leaving the sample reservoir after the seal is formed, or from evaporating from the sample reservoir. In one embodiment, the sealing member contacts the floor of the lower compartment. In one embodiment, the seal is an O-ring and makes contact with the floor of the lower chamber around the entire circumference of the reservoir, thereby sealing the reservoir. In one embodiment, the plunger in the lower position also closes the sample outlet and prevents fluid contained in the test compartment from reentering the lower chamber.

The "sample collection well" is where fluid sample is gathered in preparation for analysis. In one embodiment, the sample collection well is generally cylindrical in its shape, but in other embodiments it can assume any shape consistent with its function. The sample collection well has upper and lower chambers. In one embodiment, the upper chamber is the area above the expression plate, and the lower chamber is the area below the expression plate. An "expression plate" refers to a surface where a sample applicator filled with fluid sample can be squeezed or crushed against to express sample from the applicator. The expression plate can have openings or holes to allow the passage of expressed fluid sample to the sample collection well. The expression plate can be located within the sample collection well, but can also be placed in another location where expressed sample will flow to the collection well. In one embodiment, the expression plate is located in the sample collection well and divides the upper and lower chambers, and has one or more holes or openings through which fluid sample can flow from the upper chamber to the lower chamber. When the sample applicator is pressed against the expression plate, sample flows through the opening in the expression plate, and into the lower chamber. The term "plunger" refers to a part of the device that moves through a portion of the device to bring two parts into contact and perform a function in the operation of the device. In one embodiment the plunger moves within a circular or cylindrical part of the device, and resembles a piston. In one embodiment, the plunger moves through the sample collection well.

In one embodiment, the plunger has one or more plunger arms extending upward. In one embodiment, the one or more plunger arms extend upward inside the sample collection well and beside the expression plate. In one embodiment, the bottom of the cap interacts with the plunger arms to move the plunger from the raised position to the lower position when the cap is applied to the sample collection well. The sample reservoir can be located at the base of the sample collection well. In one embodiment, the cap and the sample well have complementary engaging screw threads. Furthermore, when the screw threads are fully engaged, the plunger may be in the lower position.
By "fully engaging" screw threads is meant that the cap has been turned onto the sample collection well and the complementary screw threads engaged to the extent that the cap is hand tight. The sample outlet provides for fluid communication between the lower chamber and the test compartment.

The "test element" can be any element that provides a detectable result. In some embodiments, the test element is a test strip. In some embodiments, the test strip has specific binding molecules immobilized on the test strip and reagents for performing an immunoassay, but in other embodiments the test strip has a chemical test, but can also have reagents necessary to conduct any suitable test that provides a detectable result. In one embodiment, the test element contains reagents for detecting the presence of a drug of abuse.

The sample can be any fluid sample. Examples of fluid samples include oral fluid, saliva, blood, serum, plasma, urine, feces, spinal fluid, vaginal swabs, mucus, and tissue. "Saliva" refers to the excretions of the salivary glands. "Oral fluid" is any fluid present in the buccal cavity. Where the analyte to be detected is a drug of abuse, the saliva of the test subject will contain sufficiently high concentrations of the drug for testing if the test subject has recently ingested the drug.

The analyte can be any analyte for which a test element is available, or can be developed. Examples of analytes of interest include a drug (such as a drug of abuse), a hormone, a protein, a peptide, a nucleic acid molecule, an etiological agent, and a specific binding pair member. A "drug of abuse" (DOA) is a drug that is taken for non-medicinal reasons (usually for mind-altering effects). The abuse of such drugs can lead to physical and mental damage and (with some substances) dependence, addiction and even death. Examples of DOAs include cocaine; amphetamines (e.g., black beauties, white bennies, dextroamphetamines, dexies, beans); methamphetamines (crank, meth, crystal, speed); barbiturates (Valium®, Roche Pharmaceuticals, Nutley, New Jersey); sedatives (i.e. sleep-aids); lysergic acid diethylamide (LSD); depressants (downers, goofballs, barbs, blue devils, yellow jackets, ludes); tricyclic antidepressants (TCA, e.g., imipramine, amitriptyline and doxepin); phencyclidine (PCP); tetrahydrocannabinol (THC, pot, dope, hash, weed, etc.); and opiates (e.g., morphine, opium, codeine, heroin, oxycodone).

In the kit of the present invention, the sample applicator can comprise an absorbent member and a handle. The absorbent member can be a sponge, a foam, or any material suitable for absorbing fluids. In one embodiment, the absorbent member is treated with a solution that stimulates salivation in a test subject. The kit can also include instructions for using the device. In one embodiment, the instructions are for using the device to determine the presence of an analyte in oral fluid or saliva. The method of the invention can also include the step of moving the plunger to the lower position to seal an aliquot of fluid sample in the reservoir. In one embodiment, the sample is applied to the sample applicator by placing the sample applicator into the mouth of a test subject, whereby the sample applicator is filled with the saliva of the test subject. The liquid sample can be applied to the test device by pressing the sample applicator against the expression plate of the device, and wringing or squeezing the sample applicator out so that liquid sample flows into the sample collection well.

The method can also include fully engaging the screw threads of the cap and sample collection well, and thereby lowering the plunger to the lower position and sealing an aliquot of sample in the reservoir. In another embodiment, the method includes the step of placing the cap on the sample collection well and thereby lowering the plunger to the lower position and sealing an aliquot of sample in the reservoir.

The present invention includes a variety of other useful aspects, which are detailed herein. These aspects of the invention can be achieved by using the articles of manufacture and compositions of matter described herein. With reference to the present disclosure, it will be further recognized that various aspects of the present invention can be combined to make desirable embodiments of the invention. In addition, a variety of other aspects and embodiments of the present invention are described herein.

The summary of the invention described above is not limiting and other features and advantages of the invention will be apparent from the following detailed description, as well as from the claims.

### Brief Description of the Drawings

Figure 1 provides a perspective view of one embodiment of the present invention.

Figure 2 provides an exploded view of the device of Figure 1.

Figure 3 provides another exploded view of the device of Figure 1.

Figure 4 show all six sides of the device of Figure 1.

Figure 5 shows top, side, cut-away and perspective views of the sample application well **120.**

Figure 6 provides an exterior view and a cut-away view of the device of Figure 1, illustrating the state of the device prior to use. Downward pointing arrows illustrate the future path of expressed fluid into the lower chamber **330.**

Figure 7 provides an exterior view and a cut-away view of the device of Figure 1, illustrating the state of the device during the expression of the sample **710** from the absorbent member **160.**

Figure 8 an exterior view and a cut-away view of the device of Figure 1, illustrating the state of the device prior to sealing the lower chamber **330.**

Figure 9 an exterior view and a cut-away view of the device of Figure 1, illustrating the state of the device after the device has been capped.

### Detailed Description

In the following detailed description, reference is made to the accompanying drawings that form a part hereof, and in which is shown by way of illustration specific embodiments in which the invention may be practiced. It is understood that other embodiments may be utilized and structural changes may be made without departing from the scope of the present invention.

One aspect of the present invention is a test device for testing a fluid sample for the presence of an analyte. The devices also allow a quantity of sample to be easily stored for confirmatory testing later in a reservoir on the device. If desired, a different principle of testing can be used in the confirmatory test. The confirmation sample is therefore safely stored from Contamination. The confirmatory sample is easily sequestered during normal assay procedures, and without any additional steps that need to be taken. In one embodiment, when the user caps the device, a confirmation sample is automatically sequestered in a reservoir for future testing as the application of the cap lowers a seal over the reservoir.

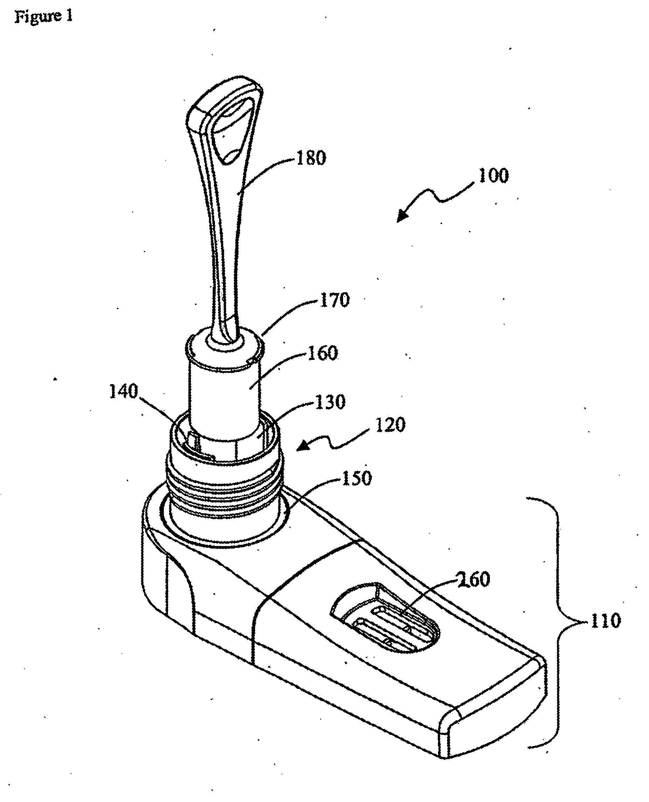

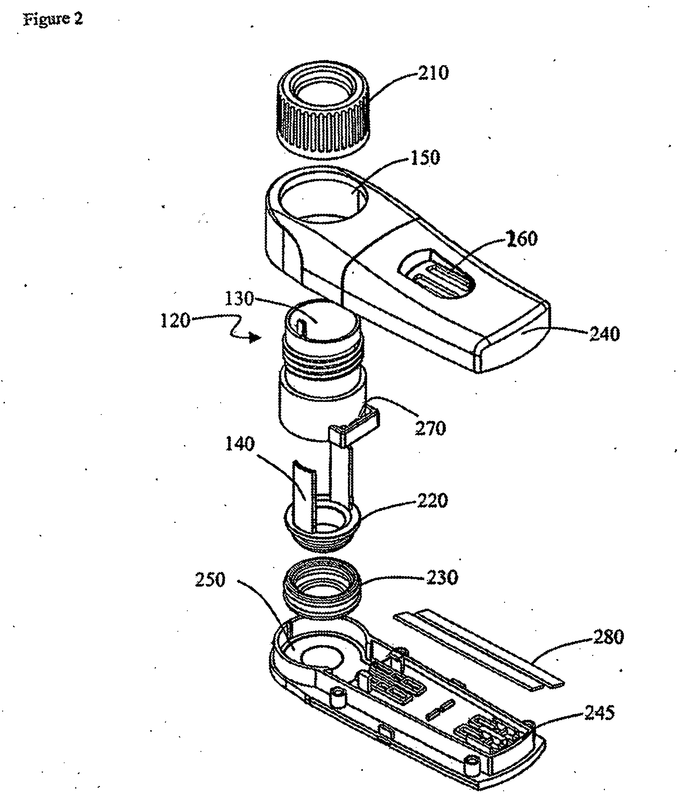

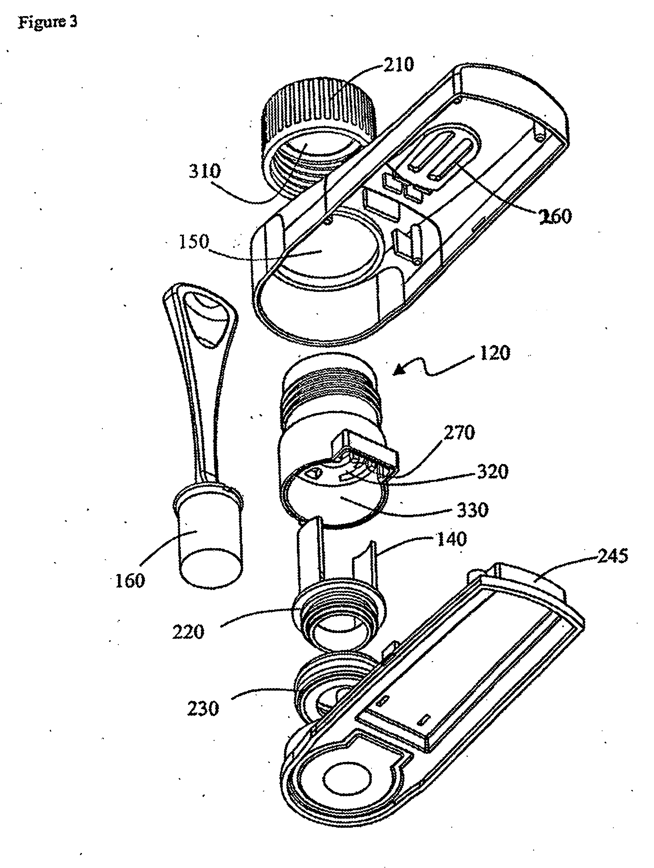

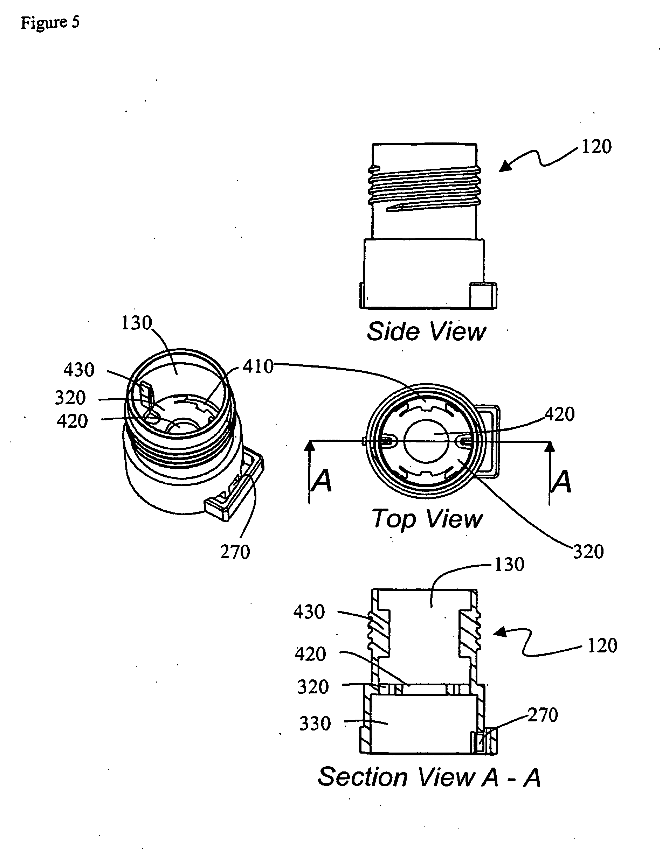

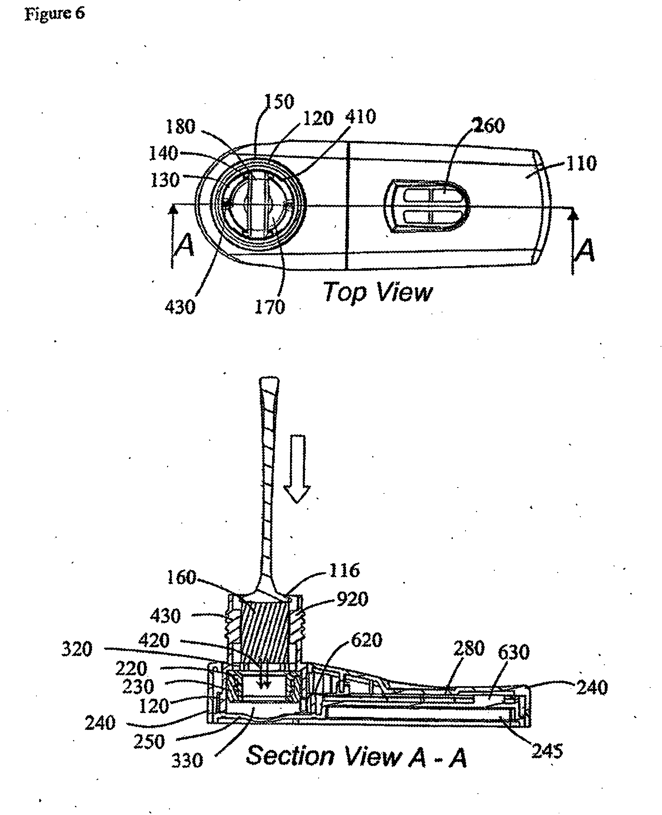

Figures 1 though 9 illustrate one embodiment of the present invention, a test device 100 for detecting an analyte suspected of being present in a fluid sample. As shown in Figure 1 through 3, the test device has a casing **110** and a sample collection well **120** (see Figure 5). The casing is composed of a top **240** and a base **245.** The casing has a port **150,** into which the sample collection well interfaces with the casing and the test compartmen (**630,** Figure 6). The test compartment contains at least one test element **280,** such as a test strip. In this embodiment, the top of the casing has a window 260 through which the test remits on the test strips can be viewed. The sample collection well is situated in the port and has an upper chamber **130,** an expression plate **320**, a lower chamber **330,** and a sample outlet **270,** which provides fluid communication between the sample collection well and the test compartment. The fluid communication is provided when the plunger is in the raised position, and no fluid communication occurs when the plunger is in the lower position.

The expression plate has one or more openings **420** through which fluid can flow. The expression plate can also have an opening sized and shaped so that fluid can be removed from the lower chamber by a pipette, syringe or other convenient mesas. In this embodiment, the expression plate is located in the sample collection well, dividing the upper chamber from the lower chamber. But in other embodiments the placement of the expression plate can vary.

A reservoir **250** is located within or below the lower chamber and can store a fluid sample for confirmation testing. In certain embodiments, the reservoir is in vertical alignment with the port. In one embodiment, the reservoir is an indentation (shown here) in the lower surface of the sample collection well. In other embodiments, the reservoir can be a chamber or other storage area receiving fluid sample from the sample collection well. In the embodiment illustrated, the reservoir is located at the bottom of the semple collection well, in other embodiments, it can be located in the lower end of the sample collection well, or even in another location.

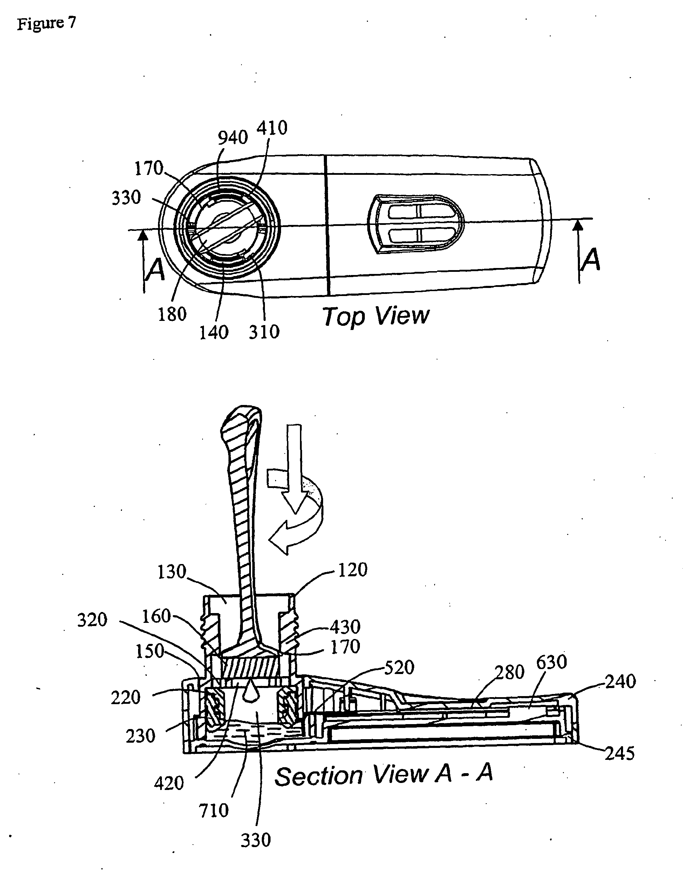

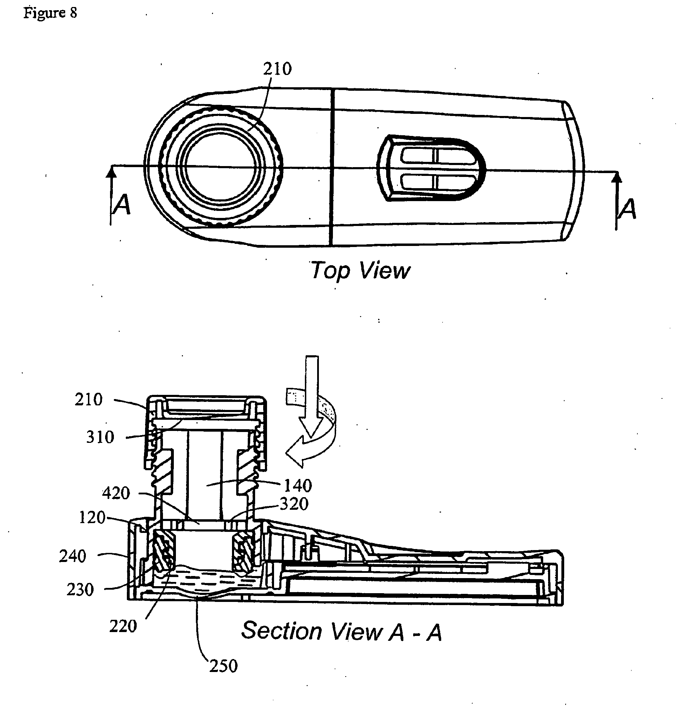

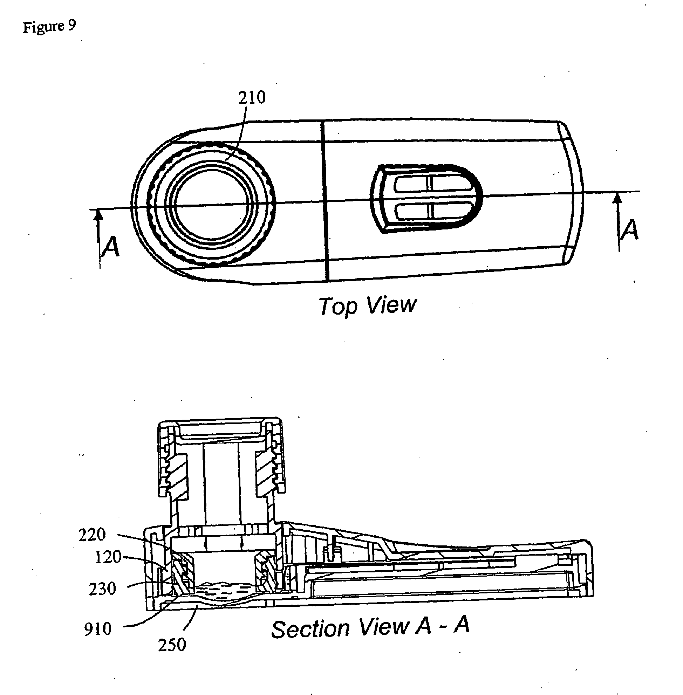

The sample collection well also has a plunger **220** having both raised and lowered positions within the sample collection chamber (see Figures 6 through 9). In one embodiment, the plunger has a seal **230** attached to its lower end. When the plunger is in the raised position, the lower chamber and the test compartment are in fluid communication with each other through the sample outlet, and fluid may flow freely from the lower chamber of the sample collection well and into the test compartment, through the sample outlet (see Figures 7 and 8). When the plunger is in the lower position, the seal fits over the reservoir (or opening thereto), and has a contact point 910 with the reservoir, and seals the lower chamber from the reservoir. Furthermore, when the plunger is in the lower position, the lower chamber is also sealed from the test compartment (Figure 9) by the closing of the sample outlet. When the lower chamber is sealed from the test compartment, the lower chamber and the test compartment are no longer in fluid communication with each other and fluid does not flow from the lower chamber into the test compartment. In other embodiments, the seal can be present as a separate piece that is moved by movement of the plunger. For example, the seal can be present at the bottom of the sample collection cup. In one embodiment, the seal can be an O-ring present around the aperture into the reservoir, and closed by the plunger being lowered into it. In another embodiment, the seal can be a separate piece existing above the aperture of the reservoir, and pressed by the plunger to form the seal around the reservoir when the plunger is moved to the lower position.

The test device also has a cap **210** for the sample collection well. In one embodiment, the cap and the sample well have complementary engaging screw threads. In the embodiment shown, the cap is adapted so that a portion of the cap **310** interacts with the plunger to move the plunger from the raised position to the lowered position when the cap is applied to the sample collection well. The interaction can be physical contact between the parts, where contact with the cap transfers force to the plunger and causes the plunger to move to the lower position. In one embodiment, the bottom of the cap interacts with the plunger to apply the force as the cap is screwed onto the sample-collection well. When the cap is screwed onto the sample collection well, the screw threads are fully engaged and the plunger has moved to the lower position, and the test compartment and the lower chamber are no longer in fluid communication. Additionally, the sample outlet provides for fluid communication between the lower chamber and the test compartment when the sample outlet is open. While a screw cap is used in some embodiments, other types of caps can also be used. For example, the cap can snap onto the sample collection well and thereby press down the plunger. The cap could also fit into the sample collection well in the style of a cork and therefore be wedged into the sample collection well, meaning that it is held in the well by the expansive forces of the cap. Any format that serves the purpose of applying force to the plunger while sealing the opening of the sample collection well is suitable.

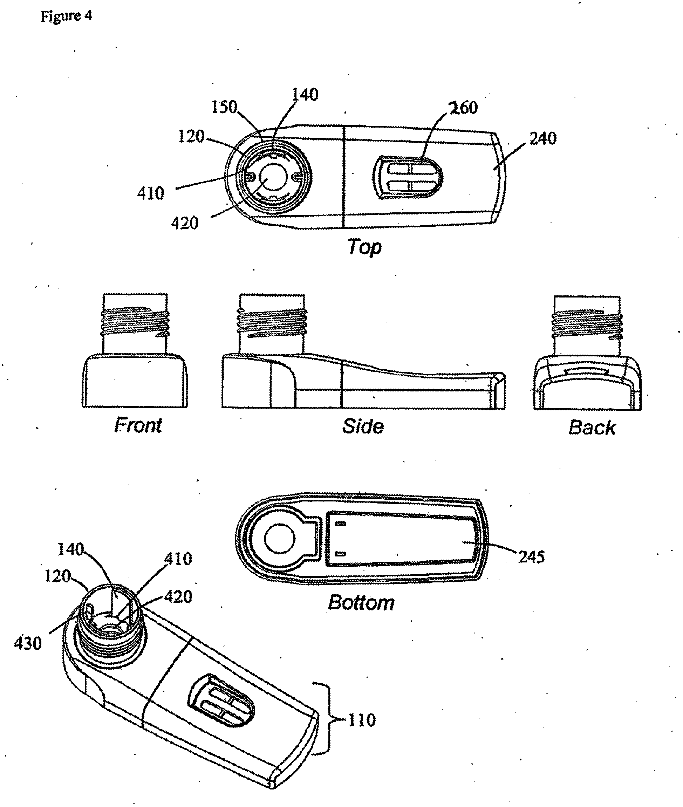

In a further embodiment, the plunger has one or more plunger arms 140 which extend upward from the direction of the base of the plunger. As illustrated in Figure 4, the plunger arms may also extend upward inside the sample collection well and beside or through the expression plate. In the embodiment shown, slots **410** have been provided in the expression plate to accommodate the plunger arms. A portion of the cap (e.g., the bottom of the cap) interacts with the plunger arms to move the plunger from the raised position to the lowered position when the cap is applied to the sample collection well. As previously discussed, when the cap is screwed onto the device, the reservoir is sealed, and the sample outlet is closed such that the lower chamber and the test compartment no are longer in fluid communication.

### Sample Applicator

A sample applicator may be supplied with the device of the present invention. In general, sample applicators consist of an absorbent member **160** and a handle **180.** The absorbent member can be made of medical grade sponge or foam material commonly used in the art. But many other materials are available for use as an absorbent member, such as cotton or paper, or any material having suitable absorbent capacity. The handle is generally rigid, to facilitate manipulation of the absorbent member. The handle may be made of any material commonly employed in the art, such as plastic, wood, metal or cardboard. In one embodiment, the handle has a rim **170** to which the absorbent member is attached.

In some embodiments of the present invention, the upper chamber of the sample collection well may have vertical ribs **430** (Fig. 4). The applicator can be adapted so that its rim locks under the ribs when the applicator has been sufficiently pressed against the expression plate and then twisted to fit under the ribs. This may ensure that the absorbent member of the applicator has been sufficiently squeezed to express as much sample contained therein as possible.

### Test Strips

A variety of test components can be incorporated into the present invention, and many varieties of test components are known in the art. This discussion provides only a brief overview of some embodiments. One type of test component is a test strip. Analyte test strips are test components provided in a variety of formats, such as immunoassay or chemical test format, for detecting analytes of interest in a sample, such as a drug of abuse or a metabolite suggestive of health status. Test components or test strips can be provided in either noncompetitive or competitive assay formats. In some formats, the test strips have a bibulous material having a sample application zone, a reagent zone and a test result zone. The sample is applied to the sample application zone and flows into the reagent zone by capillary action. In the reagent zone, the sample dissolves and mixes with reagents necessary for detection of the analyte (if it is present in the sample). The sample, now carrying the reagents, continues to flow to the test results zone. Additional reagents are immobilized in the test result zone, such as a specific binding molecule for the analyte. These reagents react with and bind the analyte (if present) or one of the first reagents from the reagent zone.

Normally, in noncompetitive formats, a signal is produced if the sample contains the analyte, and no signal is produced if the analyte is not present. In competitive formats, a signal may be produced if no analyte is present, and no signal if analyte is present. Of course how the results are interpreted depends on the design of the assay, and persons of ordinary skill in the art are familiar with test strip designs.

When the test element is a test strip, it may be made of bibulous or non-bibulous material. A test strip can include more than one material, which are then in fluid communication. One material of a teat strip may be overlaid on another material of the test strip, such as for example, filter paper overlaid on nitrocellulose. Alternatively or in addition, a test strip may include a region comprising one or more materials followed by a region comprising one or more different materials. In this case, the regions are in fluid communication and may or may not partially overlap one another. The material or materials of the test strip can be bound to a support or solid surface such as a supporting sheet of plastic, to increase its handling strength.

In embodiments where the analyte is detected by a signal producing system, such as by one or more enzymes that specifically react with the analyte, one or more components of the signal producing system can be bound to the analyte detection zone of the test strip material in the same manner as specific binding members are bound to the test strip material, as described above. Alternatively or in addition, components of the signal producing system that are included in the sample application zone, the reagent zone, or the analyte detection zone of the test strip, or that are included throughout the test strip, may be impregnated into one or more materials of the test strip. This can be achieved either by surface application of solutions of such components or by immersion of the one or more test strip materials into solutions of such components. Following one or more applications or one or more immersions, the test strip material is dried. Alternatively or in addition, components of the signal producing system that are included in the sample application zone, the reagent zone, or the analyte detection zone of the test strip, or that are included throughout the test strip, may be applied to the surface of one or more test strip materials of the test strip as was described for labeled reagents.

The zones can be arranged in many formats, for example, sample application zone, one or more reagent zones, one or more test results determination zones, one or more control zones, one or more adulteration zones, and fluid absorbing zone. If the test results determination zone includes a control zone, preferably it follows the analyte detection zone of the test result determination zone. All of these zones, or combinations thereof, can be provided in a single strip of a single material. Alternatively, the zones are made of different materials and are linked together in fluid communication. For example, the different zones can be in direct or indirect fluid communication. In this instance, the different zones can be jointed end-to-end to be in fluid communication, overlapped to be in fluid communication, or be communicated by another member, such a joining material, which is preferably bibulous such as filter paper, fiberglass or nitrocellulose. In using a joining material, a joining material may communicate fluid from end-to-end joined zones or materials including such zones, end-to-end joined zones or materials including such zones that are not in fluid communication, or join zones or materials that include such zones that are overlapped (such as but not limited to from top to bottom) but not in fluid communication.

When and if a test strip includes an adulteration control zone, the adulteration control zone can be placed before or after the results determination zone. When a control zone is present in the results determination zone on such a test strip, then the adulteration control zone is preferably before the control zone, but that need not be the case. In the embodiment of the present invention where a test strip is a control test strip for the determination of an adulteration analyte and/or a control, then the adulteration control zone can be placed before or after the control zone, but is preferably before the control zone. The adulteration zone provides a signal if there is present in the sample an adulterant being tested for to determine if an attempt has been made to defeat the purpose of the assay by the test subject.

Any analyte can be tested for utilizing the present invention and a suitable test element. In particular, the present invention can be utilized for the detection of a drug of abuse in saliva or oral fluid. Additional examples of analytes to be tested for include but are not limited to creatinine, bilirubin, nitrite, protein (nonspecific), hormones (e.g. human chorionic gonadotropin, luteinizing hormone, follicle stimulating hormone, etc.), blood, leukocytes, sugar, heavy metals or toxins, bacterial components (e.g. proteins or sugars specific to a particular type of bacteria, such as *E. coli*0157:H7, *S. aureus, Salmonella, C. perfringens, Campylobacter, L. monocytogenes, V. parahaemolyticus,* or *B.cereus*) and physical characteristics of the urine sample, such as pH and specific gravity. Any other clinical urine chemistry analyte that can be adapted to a lateral flow test format may also be incorporated into the present device.

### Types of Samples

Any sample type can be tested with the device of the present invention including liquids of biological origin (e.g., body fluids and clinical samples). Liquid samples may be derived from solid or semi-solid samples, including feces, biological tissue, and food samples. Such solid or semi-solid samples can be converted into a liquid sample by any suitable method, for example by mining chopping, macerating, incubating, dissolving or enzymatically digesting solid samples in a suitable liquid (e.g., water, phosphate-buffered saline, or other buffers). "Biological samples" include samples derived from living animals, plants, and food, including for example urine, saliva, blood and blood components, cerebrospinal fluid, vaginal swabs, semen, feces, sweat, exudates, tissue, organs, tumors, tissue and organ culture, cell cultures and conditioned media therefrom, whether from humans or animals. A preferred biological sample is urine. Food samples includes samples from processed food components or final products, meat, cheese, wine, milk and drinking water. Plant samples include those derived from any plant, plant tissue, plant cell cultures and conditioned media therefrom. "Environmental samples" are those derived from the environment (e.g., a water sample from a lake or other body of water, effluent samples, soil samples, ground water, ocean water, and runoff water. Sewage and related wastes can also be included as environmental samples.

### Methods of Use

Another aspect of the present invention is a method of detecting an analyte suspected of being present in a liquid sample, as described above. One embodiment is shown in Figure 6. Note the position of the sample applicator in the top view and the section view below. In use, the applicator is fitted within the upper chamber of the sample well and comes to rest against the expression plate. At this point the plunger, with its attached seal, is in its raised position. The lower chamber is in fluid communication with the test compartment because fluid entering the lower compartment can flow through the sample outlet and into the test compartment.

In some embodiments of the present invention, a small piece of wicking paper 620 is placed between the sample application zone of the test strips and the sample outlet. This facilitates sample flow from the sample collection well through the sample outlet and to the test strips. Any type of bibulous material that supports capillary flow can be used as the wicking paper. For example, a small piece of glass fiber paper treated with buffer and detergent to make it hydrophilic can be used, or polyester fiber, also treated to make it more hydrophilic.

The wicking paper can be used to adjust the characteristics of the sample before it is loaded onto the test strip. For example, saliva might contain an antibody that cross-reacts with one of the anti-drug antibodies (if the presence of a drug is being tested for). Thus, an antibody (a scavenger antibody) which reacts with the saliva antibody, and thereby inactivates it, can be affixed to the wicking paper. When saliva is extremely viscous, therefore slowing the wicking of the sample through the test strips, buffers, detergents and proteases can be included in the wicking paper to denature and cleave the proteins in the saliva, thereby rendering it less viscous.

In one embodiment of the present invention, the sample is applied to the sample applicator by placing the sample applicator into the mouth of a test subject, where the absorbent member becomes filled with saliva. The applicator can also be placed into a beaker or tube of previously collected sample, until the absorbent member is saturated. If a sample was previously collected and is stored in another container, such as a tube or cup with a lid, it is also acceptable to pipette a small amount of sample, similar to the amount absorbed by an absorbent member, into the sample well. In certain embodiments, about 100 ul to about 250 ul of sample can be pipetted into the sample well. But the amount of fluid sample applied to the sample collection well can be any appropriate amount such as, for example, from about 250-500 ul, or from about 500-750 ul, or about 1 ml. "About" means plus or minus 10%.

Figure 7 illustrates liquid sample applied to the test device by pressing the sample applicator against the expression plate of the device, and wringing the sample applicator out so that liquid sample flows into the bottom chamber of the sample collection well. Note in this embodiment that the applicator has been pressed down and twisted so that the rim of the applicator locks under the flanges 430. The expressed sample 710 has passed through the orifice in the expression plate and has collected in the lower chamber. The plunger and seal are still in the raised position. The wicking paper is in contact with the expressed sample that has passed through the sample outlet and sample is wicked to the test strips by the wicking paper beginning the assay.

In a further embodiment, the cap and the sample collection well have complementary engaging screw threads (see Figures 8 and 9). These embodiments include the step of fully engaging the screw threads of the cap and sample collection well, and thereby lowering the plunger to the lower position and sealing an aliquot of sample in the reservoir (which is an indentation at the bottom of the lower chamber in this embodiment). In other embodiments, the method includes the step of placing the cap on the sample collection well (e.g., by snapping or pressing it on) and thereby lowering the plunger to the lower position and sealing an aliquot of sample in the indentation. Figure 8 shows the beginning of putting the cap on the test device. The sample applicator has been removed and thrown away. The plunger and seal are still in the raised position, but the tops of the arms on the plunger are touching the bottom surface of the cap. Also, the wicking paper and the test compartment are still in fluid communication with the lower chamber.

Next, the cap is screwed onto the collection well and the screw threads are completely engaged. As shown in Figure 9, the cap has pushed on the arms of the plunger, forcing the plunger and its attached seal into the lowered position. Note that the seal makes contact with the indentation (see point 910). When this happens, the sample remaining in the lower chamber is sealed off from the test compartment. Additionally, the test compartment is no longer in fluid communication with the rest of the device, especially the lower chamber.

### Test Kits

Test kits of the invention can be packaged in a variety of formats, depending upon the customer's needs. For example, a facility that conducts large numbers of pre-employment drug screenings may prefer boxes of 1 set of instructions plus 20 vacuum packed set of devices and applicators. On the other hand, another user may prefer boxed kits that contain only one device, one sample collector, and one set of instructions. Any suitable packaging materials can be used. Test kits may be packaged in a box, or may be wrapped in protective material such as cellophane. Test kits may also be provided with a test device (and applicator) provided in the same or separate sealed pouches, which may or may not then be provided in box or other packaging.

### Examnle 1 - Pre-Employment Drug Screening

The devices of the invention can be utilized in a variety of contexts, for example, for pre-employment drug screening. The person to be tested provides a sample of oral fluid by placing the sample applicator into his or her mouth, and allowing it to remain in the mouth for about 5 minutes. In embodiments for pre-employment drug screening the device contains test strips for several common drugs of abuse, in this embodiment cocaine, methamphetamine, phencyclidine, THC, morphine, and amphetamines. These test strips utilize a competitive immunoassay format where labeled specific binding molecules (antibodies in this embodiment) for each drug being tested are present on the label zone (or reagent zone) of the test strip. The test lines contain the antigen being tested for. If analyte is present in the sample it is bound by labeled specific binding molecules in the label zone, thereby preventing the labeled antibody from binding to the test line. Thus, no signal occurs on the test line when analyte is present. Conversely, when no antigen is present in the saliva, the labeled antibodies bind to the test line providing the signal on the test line.

After receiving the filled or soaked sample applicator, the testing technician inserts it into the sample collection well of the device and presses the applicator down into the expression plate and then twists it, to lock the rim of the applicator under a pair a flanges (provided in this embodiment). Saliva is thereby expressed from the absorbent foam of the sample applicator and flows through holes in the expression plate and into the lower chamber of the sample collection well. Since the plunger is in the first position, sample also flows through the sample outlet and into the test compartment. When sample flows into the lower chamber of the sample collection well, it comes into contact with wicking paper extending into the lower chamber and flows out the sample outlet and to the test strips, thereby initiating the assays. When all of the sample is loaded, the applicator is discarded, and the cap is screwed onto the sample application well. When the cap is screwed onto the device, the force applied by the cap pushes the plunger into the lower position. As the plunger moves into the lower position, the seal is lowered around the reservoir and an aliquot of fluid sample is sequestered in the reservoir. When the plunger is lowered, the sample outlet is also closed, stopping the flow of any additional sample to the test compartment. After about 3-5 minutes, the control indicia are provided indicating that the assay is complete. A signal is provided at the test lines for each drug being tested for, indicating that no drugs of abuse are present in the saliva sample (this assay is in a competitive format). If a positive result is determined, the device may be sent to a confirmatory laboratory so that the aliquot of sample preserved in the reservoir can be tested to confirm the result.

The invention illustratively described herein may be practiced in the absence of any element or elements, limitation or limitations that are not specifically disclosed herein. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by various embodiments and optional features, modification and variation of the concepts herein disclosed may be resorted to by those skilled in the art, and that such modifications and nations are considered to be within the scope of this invention as defined by the appended claims.

## Claims

1. A test device (100) for detecting an analyte suspected of being present in a fluid sample, comprising:
a casing (110), comprising a port (150) for a sample collection well (120);
a test compartment (630) containing at least one test element (280);
a sample collection well (120) situated in the port (150) and comprising an upper chamber (130), an expression plate (320), a lower chamber (330), and a sample outlet (270), **characterised by**
a reservoir (250) within or below the lower chamber (330) for storing fluid sample for confirmation testing;
a plunger (220) comprised within the sample collection well (120), the plunger (220) having a raised position and a lower position;
a seal (230) that fits sealably over the reservoir (250) when the plunger (220) is located in the lower position;
a cap (210) for the sample collection well (120), wherein a portion (310) of the cap (210) interacts with the plunger (220) to move the plunger (220) from the raised position to the lower position when the cap (210) is applied to the sample collection well (120),
wherein, when the plunger (220) is in the raised position, the test compartment (630) and lower chamber (330) are in fluid communication and, when the plunger (220) is in the lower position, the test compartment (630) and the lower chamber (330) are not in fluid communication.

2. The device (100) of claim 1, wherein the plunger (220) further comprises one or more plunger arms (140) extending upward.

3. The device (100) of claim 2, wherein the one or more plunger arms (140) extend upward inside the sample collection well (120) and beside the expression plate (320).

4. The device (100) of claim 3, wherein the portion (310) of the cap (210) interacts with the plunger arms (140) to move the plunger (220) from the raised position to the lower position when the cap (210) is applied to the sample collection well (120).

5. The device (100) of any preceding claim, wherein the reservoir (250) is an indentation located in the base of the sample collection well (120).

6. The device (100) of any preceding claim, wherein the cap (210) and the sample collection well (120) comprise complementary engaging screw threads.

7. The device (100) of claim 6, wherein when the screw threads are fully engaged, the plunger (220) is in the lower position.

8. The device (100) of any preceding claim, wherein the sample outlet (270) provides for fluid communication between the lower chamber (330) and the test compartment (630).

9. The device of any preceding claim, wherein the test element (280) is a test strip.

10. The device of claim 9, wherein the test strip comprises specific binding molecules immobilized on the test strip.

11. The device of claim 9, wherein the test strip comprises a chemical test.

12. The test device (100) of any preceding claim, wherein the plunger (220) has a seal (230) at the lower end that fits sealably over the reservoir (25) when the plunger (220) is located in the lower position.

13. A kit comprising:
a test device (100) as claimed in any preceding claim; and
a sample applicator.

14. The kit of claim 13, wherein the sample applicator comprises an absorbent member (160) and a handle (180).

15. The kit of claim 14, wherein the absorbent member (160) is a sponge or a foam.

16. The kit of claim 13 or claim 14, wherein the absorbent member (160) is treated with a solution that stimulates salivation in a test subject.

17. The kit of claim 13, 14 or 15, further comprising instructions for using the device to determine the presence of an analyte in saliva.

18. A method of detecting an analyte suspected of being present in a liquid sample, comprising
applying a liquid sample suspected of containing the analyte to a sample applicator;
applying the liquid sample to a test device (100) as claimed in any one of claims 1 to 12 by wringing the sample applicator into the test device; and determining if the analyte is present in the sample.

19. The method of claim 18, wherein sample is applied to the sample applicator by placing the sample applicator into the mouth of a test subject.

20. The method of claim 18 or claim 19, wherein the sample applicator is filled with saliva.

21. The method of claim 18, 19 or 20, wherein liquid sample is applied to the test device (100) by pressing the sample applicator against the expression plate (320) of the device (100), and wringing the sample applicator out so that liquid sample flows into the lower chamber (330) of the sample collection well (120).

22. The method of any one of claims 18 to 21, further comprising fully engaging the screw threads of the cap (210) and sample collection well (120), and thereby lowering the plunger (220) to the lower position and sealing an aliquot of sample in the reservoir (250).

23. The method of any one of claims 18 to 22, further comprising placing the cap (210) on the sample collection well (120), lowering the plunger (220) to the lower position, and sealing an aliquot of sample in the reservoir (250).

## Patentansprüche

1. Prüfvorrichtung (100) zum Nachweisen eines Analyten, der in einer Flüssigkeitsprobe vermutet wird, umfassend:
ein Gehäuse (110), das eine Öffnung (150) für eine Probennahmevertiefung (120) umfasst;
eine Prüfkammer (630), die mindestens ein Prüfelement (280) enthält;
einen Probensammelbehälter(120), der sich in der Öffnung (150) befindet und eine obere Kammer (130), eine Ausdrückplatte (320), eine untere Kammer (330) und einen Probenauslass (270) umfasst, **gekennzeichnet durch**
einen Vorratsbehälter (250) in oder unter der unteren Kammer (330) zum Aufbewahren einer Flüssigkeitsprobe für eine Bestätigungsprüfung;
einen Tauchkolben (220), der innerhalb des Probensammelbehälters (120) vorhanden ist, wobei der Tauchkolben (220) eine angehobene und eine untere Position besitzt;
eine Dichtung (230), die dichtend über den Vorratsbehälter (250) passt, wenn sich der Tauchkolben (220) in der unteren Position befindet;
eine Abdeckung (210) für die Probennahmevertiefung (120), wobei ein Abschnitt (310) der Abdeckung (210) so mit dem Tauchkolben (220) zusammenwirkt, dass der Tauchkolben (220) aus der angehobenen Position in die untere Position bewegt wird, wenn die Abdeckung (210) auf den Probensammelbehälter (120) aufgesetzt wird,
wobei bei Positionierung des Tauchkolbens (220) in der angehobenen Position die Prüfkammer (630) und die untere Kammer (330) in Fluidverbindung stehen und, wenn der Tauchkolben (220) sich in der unteren Position befindet, die Prüfkammer (630) und die untere Kammer (330) nicht in Fluidverbindung stehen.

2. Vorrichtung (100) nach Anspruch 1, wobei der Tauchkolben (220) ferner einen oder mehrere sich nach oben erstreckende(n) Tauchkolbenarm(e) (140) aufweist.

3. Vorrichtung (100) nach Anspruch 2, wobei der eine oder die mehreren Tauchkolbenarme (140) sich im Probensammelbehälter (120) nach oben und neben der Ausdrückplatte (320) erstrecken.

4. Vorrichtung (100) nach Anspruch 3, wobei der Abschnitt (310) der Abdeckung (210) so mit den Tauchkolbenarmen (140) zusammenarbeitet, dass der Tauchkolben (220) aus der angehobenen Position in die untere Position bewegt wird, wenn die Abdeckung (210) auf den Probensammelbehälter (120) aufgesetzt wird.

5. Vorrichtung (100) nach einem der vorherigen Ansprüche, wobei der Vorratsbehälter (250) eine Vertiefung ist, die sich an der Basis des Probensammelbehälters (120) befindet.

6. Vorrichtung (100) nach einem der vorherigen Ansprüche, wobei die Abdeckung (210) und der Probensammelbehälter (120) komplementäre ineinander greifende Schraubgewinde aufweisen.

7. Vorrichtung (100) nach Anspruch 6, wobei sich der Tauchkolben (220) in der unteren Position befindet, wenn die Schraubgewinde voll im Eingriff sind.

8. Vorrichtung (100) nach einem der vorherigen Ansprüche, wobei der Probenauslass (270) eine Fluidverbindung zwischen der unteren Kammer (330) und der Prüfkammer (630) vorsieht.

9. Vorrichtung nach einem der vorherigen Ansprüche, wobei das Prüfelement (280) ein Teststreifen ist.

10. Vorrichtung nach Anspruch 9, wobei der Teststreifen spezielle Bindungsmoleküle aufweist, die auf dem Teststreifen immobilisiert sind.

11. Vorrichtung nach Anspruch 9, wobei der Teststreifen einen chemischen Test umfasst.

12. Prüfvorrichtung (100) nach einem der vorherigen Ansprüche, wobei der Tauchkolben (220) eine Dichtung (230) am unteren Ende hat, die dichtend über den Vorratsbehälter (250) passt, wenn sich der Tauchkolben (220) in der unteren Position befindet.

13. Satz, umfassend:
eine Prüfvorrichtung (100) nach einem der vorherigen Ansprüche; und
einen Probenapplikator.

14. Satz nach Anspruch 13, wobei der Probenapplikator ein Absorptionselement (160) und einen Griff (180) aufweist.

15. Satz nach Anspruch 14, wobei das Absorptionselement (160) ein Schwamm oder Schaum ist.

16. Satz nach Anspruch 13 oder Anspruch 14, wobei das Absorptionselement (160) mit einer Lösung behandelt ist, die die Speichelbildung in einer Versuchsperson stimuliert.

17. Satz nach Anspruch 13, 14 oder 15, der ferner Anweisungen zum Verwenden der Vorrichtung zum Nachweis des Vorhandenseins eines Analyten im Speichel umfasst.

18. Verfahren zum Nachweisen eines Analyten, der in einer Flüssigkeitsprobe vermutet wird, umfassend
das Auftragen einer Flüssigkeitsprobe, in der der Analyt vermutet wird, auf einen Probenapplikator;
das Auftragen der Flüssigkeitsprobe auf eine Prüfvorrichtung (100) nach einem der Ansprüche 1 bis 12 durch Ausdrücken des Probenapplikators in die Prüfvorrichtung; und
das Bestimmen, ob der Analyt in der Probe vorhanden ist.

19. Verfahren nach Anspruch 18, wobei die Probe auf den Probenapplikator durch Platzieren des Probenapplikators im Mund einer Versuchsperson aufgebracht wird.

20. Verfahren nach Anspruch 18 oder Anspruch 19, wobei der Probenapplikator mit Speichel gefüllt wird.

21. Verfahren nach Anspruch 18, 19 oder 20, wobei die Flüssigkeitsprobe durch Drücken des Probenapplikators gegen die Ausdrückplatte (320) der Vorrichtung (100) und Auswringen des Probenapplikators der Prüfvorrichtung (100) zugeführt wird, so dass die Flüssigkeitsprobe in die untere Kammer (330) des Probensammelbehälters (120) fließt.

22. Verfahren nach einem der Ansprüche 18 bis 21, das ferner das volle Eingreifen der Schraubengewinde der Abdeckung (210) und des Probensammelbehälters (120) und **dadurch** das Absenken des Tauchkolbens (220) in die untere Position und das Abdichten eines aliquoten Anteils der Probe im Vorratsbehälter (250) umfasst.

23. Verfahren nach einem der Ansprüche 18 bis 22, das ferner das Platzieren der Abdeckung (210) auf dem Probensammelbehälter (120), das Absenken des Tauchkolbens (220) in die untere Position und das Abdichten eines aliquoten Anteils der Probe im Vorratsbehälter (250) umfasst.

## Revendications

1. Dispositif d'essai (100) pour détecter un analyte suspecté d'être présent dans un échantillon de fluide, comprenant :
un boîtier (110) comprenant un orifice (150) pour puits de collecte d'échantillon (120) ;
un compartiment d'essai (630) contenant au moins un élément d'essai (280) ;
un puits de collecte d'échantillon (120) situé dans l'orifice (150) et comprenant une chambre supérieure (130), une plaque d'expression (320), une chambre inférieure (330) et un orifice de sortie d'échantillon (270), **caractérisé par**
un réservoir (250) à l'intérieur ou en dessous de la chambre inférieure (330) servant à stocker un échantillon de fluide pour effectuer un essai de confirmation ;
un piston (220) se trouvant à l'intérieur du puits de collecte d'échantillon (120), le piston (220) ayant une position haute et une position basse ;
un joint d'étanchéité (230) qui s'applique de manière étanche sur le réservoir (250) quand le piston (220) est en position basse ;
un bouchon (210) pour le puits de collecte d'échantillon (120), une partie (310) du bouchon (210) interagissant avec le piston (220) pour déplacer le piston (220) de la position haute vers la position basse quand le bouchon (210) est appliqué sur le puits de collecte d'échantillon (120),
dans lequel, quand le piston (220) est en position haute, le compartiment d'essai (630) et la chambre inférieure (330) sont en communication de fluide, et quand le piston (220) est en position basse, le compartiment d'essai (630) et la chambre inférieure (330) ne sont pas en communication de fluide.

2. Dispositif (100) selon la revendication 1, dans lequel le piston (220) comprend en outre un ou plusieurs bras de piston (140) s'étendant vers le haut.

3. Dispositif (100) selon la revendication 2, dans lequel le ou les bras de piston (140) s'étendent vers le haut à l'intérieur du puits de collecte d'échantillon (120) et à côté de la plaque d'expression (320).

4. Dispositif (100) selon la revendication 3, dans lequel la partie (310) du bouchon (210) interagit avec les bras de piston (140) pour déplacer le piston (220) de la position haute vers la position basse quand le bouchon (210) est appliqué sur le puits de collecte d'échantillon (120).

5. Dispositif (100) selon l'une quelconque des revendications précédentes, dans lequel le réservoir (250) est un renfoncement situé dans la base du puits de collecte d'échantillon (120).

6. Dispositif (100) selon l'une quelconque des revendications précédentes, dans lequel le bouchon (210) et le puits de collecte d'échantillon (120) comprennent des filetages d'engagement complémentaires.

7. Dispositif (100) selon la revendication 6, dans lequel quand les filetages sont totalement engagés, le piston (220) est en position basse.

8. Dispositif (100) selon l'une quelconque des revendications précédentes, dans lequel l'orifice de sortie d'échantillon (270) permet une communication de fluide entre la chambre inférieure (330) et le compartiment d'essai (630).

9. Dispositif selon l'une quelconque des revendications précédentes, dans lequel l'élément d'essai (280) est une bandelette réactive.

10. Dispositif selon la revendication 9, dans lequel la bandelette réactive comprend des molécules à liaison spécifique immobilisées sur la bandelette réactive.

11. Dispositif selon la revendication 9, dans lequel la bandelette réactive comprend est essai chimique.

12. Dispositif d'essai (100) selon l'une quelconque des revendications précédentes, dans lequel le piston (220) comporte un joint d'étanchéité (230) sur l'extrémité inférieure qui s'applique de manière étanche sur le réservoir (250) quand le piston (220) est en position basse.

13. Kit comprenant :
un dispositif d'essai (100) selon l'une quelconque des revendications précédentes ; et
un applicateur d'échantillon.

14. Kit selon la revendication 13, dans lequel l'applicateur d'échantillon comprend un élément absorbant (160) et une poignée (180).

15. Kit selon la revendication 14, dans lequel l'élément absorbant (160) est une éponge ou une mousse.

16. Kit selon la revendication 13 ou la revendication 14, dans lequel l'élément absorbant (160) est traité avec une solution qui stimule la salivation d'un sujet à tester.

17. Kit selon la revendication 13, 14 ou 15, comprenant en outre des instructions pour utiliser le dispositif afin de déterminer la présence d'un analyte dans la salive.

18. Procédé de détection d'un analyte suspecté d'être présent dans un échantillon liquide, comprenant :
l'application d'un échantillon liquide suspecté de contenir l'analyte sur un applicateur d'échantillon ;
l'application de l'échantillon liquide sur un dispositif d'essai (100) selon l'une quelconque des revendications 1 à 12 en essorant l'applicateur d'échantillon dans le dispositif d'essai ; et
la détermination de la présence ou non de l'analyte dans l'échantillon.

19. Procédé selon la revendication 18, dans lequel l'échantillon est appliqué sur l'applicateur d'échantillon en plaçant l'applicateur d'échantillon dans la bouche d'un sujet à tester.

20. Procédé selon la revendication 18 ou la revendication 19, dans lequel l'applicateur d'échantillon est rempli avec de la salive.

21. Procédé selon la revendication 18, 19 ou 20, dans lequel l'échantillon liquide est appliqué sur le dispositif d'essai (100) en pressant l'applicateur d'échantillon contre la plaque d'expression (320) du dispositif (100) et en essorant l'applicateur d'échantillon de manière à ce que l'échantillon liquide s'écoule dans la chambre inférieure (330) du puits de collecte d'échantillon (120).

22. Procédé selon l'une quelconque des revendications 18 à 21, comprenant en outre l'engagement total des filetages du bouchon (210) et du puits de collecte d'échantillon (120), pour ainsi abaisser le piston (220) vers la position basse et enfermer une aliquote d'échantillon dans le réservoir (250).

23. Procédé selon l'une quelconque des revendications 18 à 22, comprenant en outre le placement du bouchon (210) sur le puits de collecte d'échantillon (120), l'abaissement du piston (220) vers la position basse et l'enfermement d'une aliquote d'échantillon dans le réservoir (250).
